# NEW EUROPEAN PATENT SPECIFICATION

(11) **EP 0 556 034 B2**
(45) Date of publication and mention of the opposition decision: **21.07.2004**
(45) Mention of the grant of the patent: 11.04.2001
(21) Application number: 93300966.4
(22) Date of filing: 10.02.1993
(51) Int. Cl.: A61L 29/00, A61L 31/00

(54) **A medical instrument**
Medizinisches Gerät
Instrument médical

(30) Priority: 12.02.1992 JP 2521392; 02.02.1993 JP 1552193
(43) Date of publication of application: 18.08.1993
(73) Proprietor: DAIKYO GOMU SEIKO LTD., Tokyo (JP)
(72) Inventor: Sudo, Morihiro, Sumida-Ku, Tokyo (JP)
(74) Representative: Hayes, Adrian Chetwynd

(56) References cited:
- EP-A- 0 226 956
- EP-A- 0 384 694
- EP-A- 0 386 896
- EP-A- 0 497 567
- EP-A- 0 524 802
- DATABASE WPIL Week 9118, Derwent Publications Ltd., London, GB; AN 91-129021

## Description

This invention relates to a medical instrument.

As to medical instruments, the varieties, performances, qualities and standards of instruments or machines are provided in the Drugs, Cosmetics and Medical Instruments Laws. In this age of rapid progress of medical techniques, instruments or machines having better performance than such standard values or standard items have appeared.

As a soft elastic material for a medical instrument, there is mainly used natural rubber (NR), but at the present time, isoprene rubber (IR), butyl rubber (IIR), halogenated butyl rubbers (BIIR, CIIR), nitrile rubber (NBR), styrenebutadiene rubber (BR) have been widely used in view of these rubbers being more sanitary and have better properties.

As to synthetic resins, polyethylene (PEW), polyvinyl chloride (PVC) and polypropylene (PEW) have been authorized by the Japanese Pharmacopoeia (8th Revision) and applied to a number of medical instruments or machine parts.

PVC seems to be a clear and soft sanitary article, but detailed examination shows a problem that plasticisers such as dioctyl phthalate, dioctyl adipate (DOA) and tricresyl phosphate (TCP) or stabilisers such as zinc stearate, calcium stearate and tin compounds are dissolved out into a medical fluid. The use of PVC in the future is questionable because of the problem of protection of the environment because raw material monomers of PVC remain and poisonous gases are generated when used instruments are burnt as a waste. On the other hand, a number of studies have been made on medical instrument for preservation of blood (Japanese Patent Publication No. 42507/1990, Japanese Patent Laid-Open Publication No. 212536/1990).

Lately, PE has been widely used, since a gas sterilization method of ethylene oxide or formaldehyde is authorized and is sanitarily excellent. In particular, ultrahigh molecular weight polyethylene is relatively excellent in softening point (130°C) and laminated articles thereof with a vulcanised rubber, PP, nylon or PVC has been authorized. However, PE has a significant disadvantage in that its softening point is low.

PP has a valuable feature in its high softening point, but because of difficulty in obtaining a transparent article, it has been proposed to modify PP to give a transparent article which can be applied to medical instruments or pharmaceutical agents (Japanese Patent Laid-Open Publication Nos. 163144/1991 28246/1991).

Polyvinylidene chloride is generally blended with PVC because of the difficulty in molding an article of polyvinylidene chloride.

Fluoro resins are excellent in sanitary properties, heat resistance, acid resistance and alkali resistance, but are so inferior in adhesiveness and in workability that these resins cannot be applied to parts for widely used medical instruments.

In addition, nylons, polycarbonates, polyurethanes, polyethylene terephthalates, ethylene vinyl acetate copolymers, polystyrenes, acrylic resins, and thermoplastic elastomers, are resins each having various properties, but having many problems from the standpoint of a raw material for a medical instrument and having no property to substitute for PE or PP.

The present invention has been made with a view to developing an article for a medical instrument which is an artificial kidney device of the dialysis type using a novel material capable of dosing a patient with a medical fluid in a very sanitary manner.

The present invention provides a medical instrument which is an artificial kidney device of the dialysis type consisting of a material capable of sanitarily dosing a patient with a medical fluid, whereby the above described many problems can be resolved.

This can be attained by a medical instrument which is an artificial kidney device of the dialysis type consisting of a material containing a resin formed of or a bridged polycyclic hydrocarbon compound, as a polymeric component.

The accompanying drawings are not in accordance with the invention.

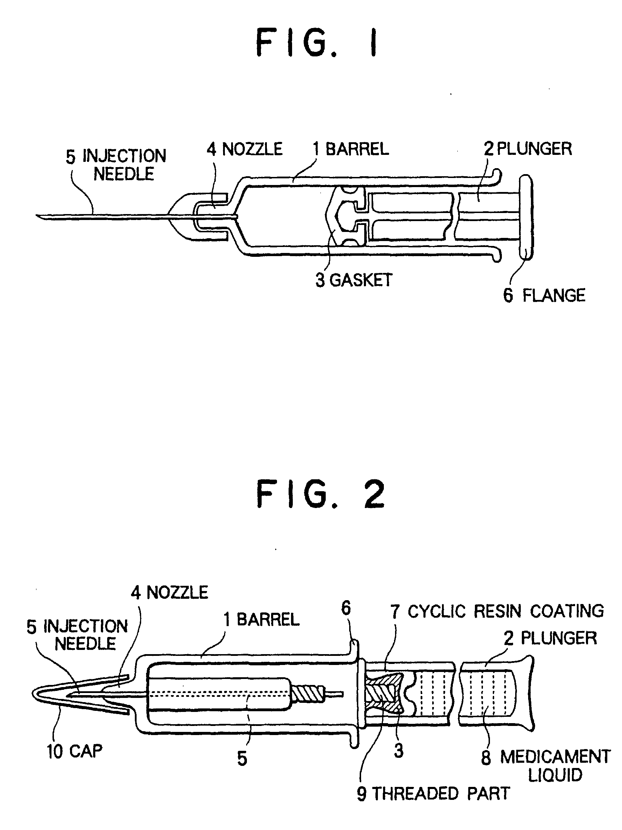

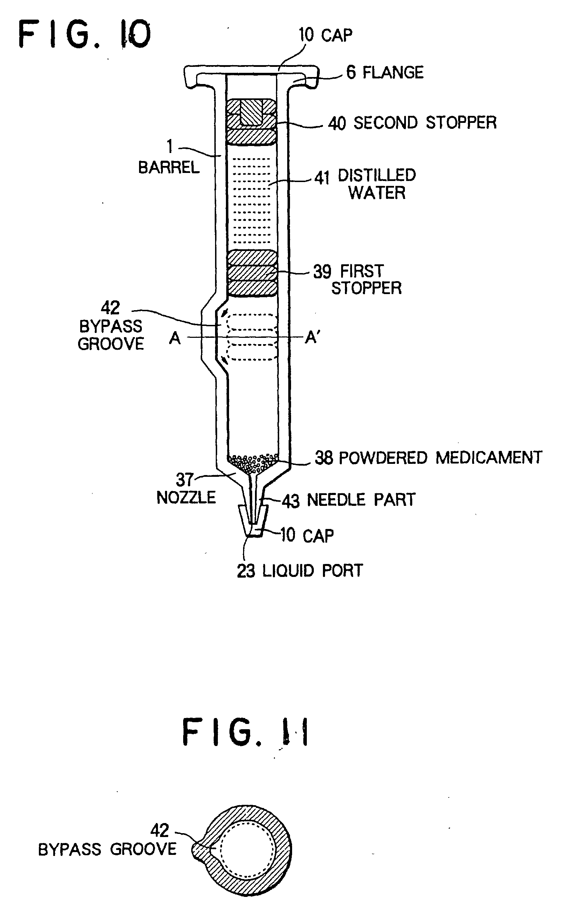

Fig. 1 is a cross-sectional view of a syringe consisting of a cyclic resin.

Fig. 2 is a cross-sectional view of a syringe-cum-container for a medical fluid, consisting of a cyclic resin.

Fig. 10 is a cross-sectional view of a syringe-cum-container filled with two kinds of drugs, consisting of a cyclic resin.

The inventors have made various studies to develop a medical instrument which is an artificial kidney device of the dialysis type and have found that a resin formed of a a bridged polycyclic hydrocarbon compound, as a polymeric component, is effective for this purpose.

Accordingly, the present invention provides a medical instrument which is an artificial kidney device of the dialysis type as defined in claim 1.

As the above described bridged polycyclic hydrocarbon compound of the present invention, there are used those having at least one unsaturated bond in the ring or substituent.

In the present invention, a resin comprising the above described bridged polycyclic hydrocarbon compound, as a polymeric component, (which will hereinafter be referred to as "cyclic resin") can contain at least one lower olefin, aromatic compound or lower olefin or aromatic vinyl monomer, as a copolymeric component, and can be a mixture with olefin resins and/or synthetic rubbers.

Furthermore, the cyclic resin of the present invention has a bromine number of at most and a softening point of at least 90°C.

In the medical instrument of the present invention, a particularly preferred embodiment includes laminated articles of the cyclic resin and other resins.

Of late, characteristic resins have been developed by new techniques of separation or purification of monomers of C₅ to C₉ fractions obtained by cracking of coal tar or napththa and polymerisation catalysts of the monomers and in particular there has been marked progress in the production of polymers of cyclic olefin monomers, in particular, bridged polycyclic hydrocarbon monomers.

The inventors have found that a resin comprising a bridged polycyclic hydrocarbon compound, as a polymeric component, is a non-crystalline material having excellent properties including alkali resistance, acid resistance, water proofness and chemical resistance, and having a high melting point, heat resistance, oxidation resistance and transparency, and is a very good resin for a medical instrument, being capable of passing the Japanese Pharmacopoeia test and being readily molded. The present invention is based on this finding.

Examples of the compound to be the polymeric component of the cyclic resin used as a medical instrument according to the present invention will be illustrated further in detail. **[0028]** As the bridged polycyclic hydrocarbon compound, it is particularly preferable to use bridged cyclic hydrocarbon compounds containing two or more rings, in particular, bridged polycyclic olefin compounds and derivatives thereof, or bridged polycyclic saturated hydrocarbon compounds having unsaturated double bonds in the substituents thereof, illustrative of which are bridged polycyclic cycloalkene compounds and lower alkyl derivatives, aryl derivatives or aralkyl derivatives thereof and vinyl derivatives, allyloxycarboxy derivatives and (meth)acryloxy derivatives of bridged polycyclic cycloalkane compounds.
bicyclo [2,2,1]-2-heptoene 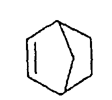 bicyclo[2,2,1]-2,5-heptodiene (2,5-norbornadiene) 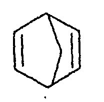 ethyl-bicyclo[2,2,1]-2-heptoene 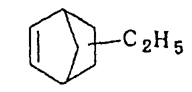 ethylidene-bicyclo[2,2,1]-2-heptoene (ethylidene-2-norbornane) 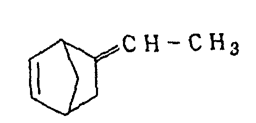 phenyl-bicyclo[2,2,1]-hepto-2-ene 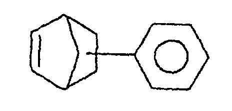 dicyclo[4,3,0]-3,8-nonadiene 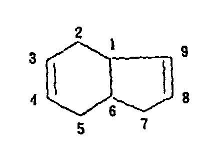 tricyclo[4,3,0,1^{2. 5}]-3-decene 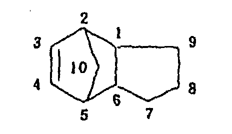 tricyclo[4,3,0,1^{2. 5}]-3,8-decene (3,8-dihydrodicyclopentadiene) 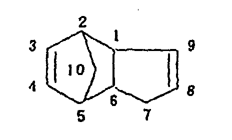 tricyclo[4,4,0,1^{2.5}]-3-undecene 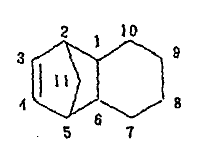 tetracyclo[4,4,0,1^{2.5}, 1^{7.10}]-3-dodecene 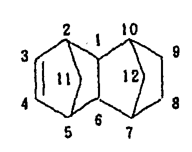 dimethyl-tetracyclo[4,4,0,1^{2. 5}, 1^{7. 10}]-3-dodecene 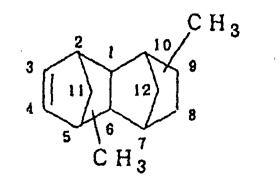 ethylidene-tetracyclo[4,4,0, 1^{2. 5}, 1^{7. 10}]-3-dodecene 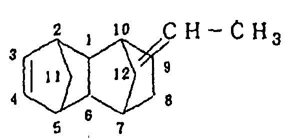 methyloxycarbonyltetracyclo[4,4,0,1^{2. 5}, 1^{7. 10}]-3-dodecene 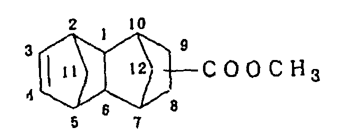 ethylidene-9-ethyltetracyclo[4,4,0,1^{2.5}, 1^{7. 10}]-3-dodecene 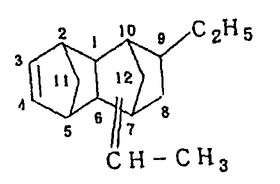 pentacyclo[4,7,0,1^{2. 5}, 0, 0^{8. 13}, 1^{9. 12}]-3- pentadecene 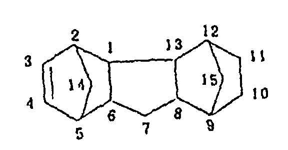 pentacyclo[6,5,1,1^{3. 6}, 0^{2. 7}, 0^{9. 13}]-4-pentadecene 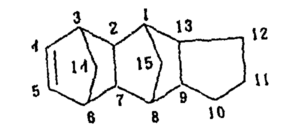 hexacyclo[6,6,1,1^{3. 6}, 1^{10. 13}, 0^{2. 7}, 0^{9.14}]-4-heptadecene 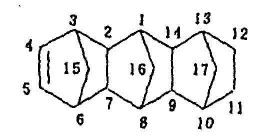 dimethyl-hexacyclo[6,6,1, 1^{3. 6}, 1^{10. 13}, O^{2. 7}, 0^{9. 14}]-4-heptadecene 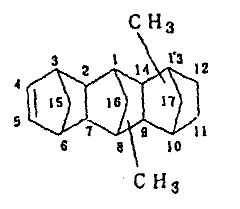 bis(allyloxycarboxy)tricyclo[4,3,0, 1^{2.5}]-decane 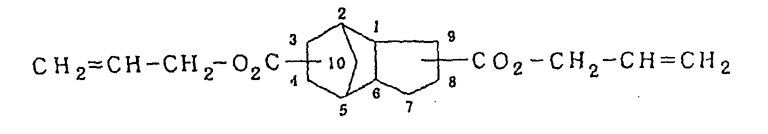 bis(metharyloxy)tricyclo[4,3,0,1^{2. 5}]-decane 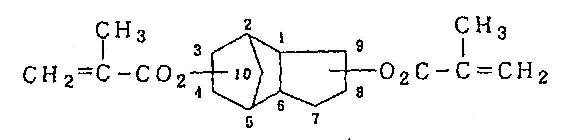 bis(acryloxy)tricyclo[4,3,0,1^{2. 5}]-decane

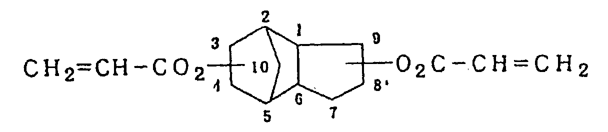

In the cyclic resin of the present invention, at least one of the above described bridged polycyclic hydrocarbon compounds is used as a polymeric component and further lower olefins, aromatic compounds or vinyl monomers of lower olefins or aromatic compounds are contained as a copolymeric component, capable of copolymerizing with these polymeric components.

Examples of these other polymeric components are ethylene, propylene, isoprene, butadiene, methylpentene, norbornene, butene and vinyltoluene.

Synthesis of the cyclic resin of the present invention can be carried out in known manner, for example, as disclosed in Japanese Patent Publications Nos. 11818/1972, 43412/1983, 1442/1986 and 19761/1987, and Japanese Patent Laid-Open Publications Nos. 75700/1975, 129434/1980, 127728/1983, 168708/1985, 115916/1986, 271308/1986, 221118/1988, 243103/1088 and 180976/1990.

Specifically, the following three types of method can be utilized:
(1) method for obtaining a bridged cyclic hydrocarbon resin, comprising subjecting a cyclopentadiene and the corresponding olefin or cyclic olefin to addition cyclization reaction (Diels Alder Reaction) to form a bridged cyclic hydrocarbon monomer, polymerising the monomer in a solvent using an aluminium compound, vanadium compound, tungsten compound or boron compound as a catalyst to form a resinous material and purifying the resin.
(2) method for obtaining the cyclic resin of the present invention, comprising polymerising a monomer to be the polymeric component of the cyclic resin of the present invention, for example, a lower alkylcycloalkene compound, cycloalkadiene compound, bridged polycyclic alkadiene compound, or bridged polycyclic alkene compound in a solvent, using e.g. a vanadium compound, aluminium compound, tungsten compound or boron compound, as a catalyst to form a high molecular weight resinous material and then hydrogenating the resinous material by the use of a nickel or platinum catalyst.
(3) method for obtaining the cyclic resin of the present invention, comprising polymerizing an acryloyl derivative of a bridged polycyclic compound by light and/or an organo peroxide to obtain a bridged cyclic resin and then purifying the resin.

In the above described three polymerisation reactions, a monomer of an aromatic compound can further be added to obtain a corresponding copolymer.

In any of the above described polymerisation methods, the presence of the monomers used as the polymeric component, low molecular weight oligomers, or metallic catalysts, in the cyclic resin of the present invention is not desired with respect to generation of odor and deterioration of the sanitariness.

Therefore, the cyclic resin of the present invention is a resin having a softening point of at least 90°C (JIS K 2207, 2531, ring and ball method).

The cyclic resin of the present invention has a bromine number of at most 1 (JIS K 2543), since if it is more than 1; coloration or discolouration takes place in a sanitary medical instrument. As a countermeasure for this coloration or discolouration, an age resistor is added.

Examples of the age resistor added to the cyclic resin of the present invention include 2-6-di-t-butyl-4-methylphenol (BHT), octadecyl-3-(4'-hydroxy-3',5'-di-t-butylphenyl) propionate (Irganox 1076 -commercial name- made by Ciba Geigy Co.), tetrakis(methylene(3,5-di-butyl-4-hydroxyphenyl) propionate]-methane (Irganox 1010 -commercial name- made by Ciba Geigy Co.), tocopehenol, 4,4'-thiobis(6-t-butyl-3-methylphenol) (Antage RC -commercial name-made by Kawaguchi Kagaku KK), bis(2,2,6,6-tetramethylpiperidyl) sebacate (Sanol LS 770 -commercial name- made by Sankyo KK), 1,3,8-triaza-7,7,9,9-tetramethyl-n-octylspiro[4,5]-decane-2,4-dione (Sanol LS 772 -commercial name-made by Sankyo KK), distearyl thiodipropionate (Antigen TPS -commercial name- made by Sumitomo Kagaku KK), pentaerythritol-tetrakis(β-lauryl-thio-propionate) (Sumilizer TPD -commercial name- made by Sumitomo Kagaku KK), 1,3,5-trimethyl-2,4,6,-tris(3,5-di-t-butyl-4-hydroxybenzyl)benzene (Ionox 330 -commercial name- made by ICI) and tris (2,4-di-t-butylphenyl) phosphite (Irganox 168 -commercial name- made by Ciba Geigy Co.).

These age resistors function to prevent the cyclic resin of the present invention from gelling by heat, light or oxygen. The amount of the age resistor to be added is generally 0.1 to 1 part by weight to 100 parts by weight of the cyclic resin and several age resistors can be used jointly.

The content of a cyclic olefin monomer in the cyclic resin of the present invention is preferably at least 30 weight % and the molecular weight of the cyclic resin is preferably 5,000 to 100,000,000. A low molecular weight resin is highly viscous, but a high molecular weight resin is powdered.

In working a resin, i.e. shaping a resin article, it is preferable to use a working aid, in particular, when the shaping operation is difficult. As the working aid, there is preferably used at least one higher fatty acid or higher fatty acid ester, silicone oil or fluorinated oil in a proportion of O to 10 weight % to 100 parts by weight of the cyclic resin.

The cyclic resin of the present invention has the following properties:

| | |
|---|---|
| Specific Gravity | 0.98-1.3 (ASTM D792) |
| Tensile Strength | 200-1000 kg/cm² (ASTM D638) |
| Tensile Elongation | 3-300 % (ASTM D638) |
| Bending Modulus | 1-50 x 10⁴ kg/cm² (ASTM D790) |
| Softening Point | 90°C or higher (ASTM D1525) |
| Transparency | 90-100 % (ASTM D1003) |
| Water Absorption Ratio | 0.01-0.1 % (ASTM D570) |
| Bromine Number | 0-1 (JIS K2543) |
| JP 12 | |
| 48 Test method of rubber stopper for transfusion | Satisfactory |
| 49 Test method of plastic container for transfusion | Satisfactory |

As described above, the cyclic resin of the present invention is an ultrahigh molecular weight resin having a high softening point, excellent physical properties such as tensile strength, high toughness, high inertness to acids or alkalis, low moisture absorption, low permeability of moisture, oxygen or air, excellent cold resistance or heat resistance, non-crystalline property and transparency.

The cyclic resin or cyclic resin composition of the present invention further contains at least one olefin type resin, illustrative of which are various polyethylenes (PEW), polypropylenes (PEW), nylons including amorphous nylon, PET, PBT, ethylene-propylene copolymers, ethylene-acrylic acid copolymers, propylene-butene copolymers, polybutenes, methylbutene copolymers, ethylene-butene copolymers, methylpentene copolymers and graft- or block copolymers of olefin type compounds.

Alternatively, the above described cyclic resin or cyclic resin composition is a mixture or blend with a synthetic rubber. Examples of the synthetic rubber are isoprene rubbers, butadiene rubbers, ethylene-propylene rubbers, ethylenepropylene-diene-terpolymers, butadiene-isoprene copolymers and isoprene-isobutylene rubbers.

When the cyclic resin of the present invention is mixed with resins or rubbers, as described above, the cyclic resin is present in a proportion of at least 30 weight % to the total composition, since if the content of the cyclic resin is less than 30 weight %, the sanitary property characteristic of the present invention, e.g. alkali resistance cannot sufficiently be given so that there is little difference from medical instruments of the conventionally used resins.

The medical instrument of the present invention can be prepared by shaping the cyclic resin composition of the present invention as it is or laminating the same with another resin. Examples of the other resin are ethylene-vinylalcohol copolymer resins (EVOH), polyvinyl alcohol (PVA), ethylenevinyl acetate copolymers or saponified products thereof (EVA), nylons including amorphous nylon, ethylene-vinyl copolymer resins, PE, PP, PET, polymethylpentenes, PVDC, acrylic resins, acrylic modified resins, ethylene-propylene copolymer resins, ethylene-butene copolymer resins and graft- or block copolymers of olefin compounds.

In the above described medical instrument of the present invention, the presence of the cyclic resin and polar group-containing resin, laminated and bonded with each other, results in improvement of the quality guarantee of a content in the container. During the same time, a good bonding can be realized by the use of a laminated layer consisting of a mixture of both the resins or with an adhesives and the sanitary property of the cyclic resin can further be improved.

For the purpose of preventing deterioration by light (ultraviolet rays, UV) or oxidation, a UV absorber or UV shielding agent can be added to a resin to be laminated. Examples of the UV absorber or UV shielding agent are p-t-butylphenyl salicylate, 2,4-dihydroxybenzophenone, 2-hydroxy-4-methoxybenzophenone, 2(2'-hydroxy-5'-methylphenyl)benzotriazole, 2(2'-hydroxy-3'-t-butyl-5'-methylphenyl)-5-chlorobenzotriazole, 2(2'-hydroxy-3',5'-di-t-butylphenyl)-5-chlorobenzotriazole, bis(2,2,6,6-dimethyl-4,4-piperidine) sebacate (Sanol LS 770 -commercial name-made by Ciba Geigy Co.), hindered amine of polymer type (Sanol LS 944 -commercial name- made by Ciba Geigy Co.), fine grain titanium oxide or zinc oxide. These UV absorbers or shielding agents can be used, individually or in combination, in a proportion of 0.01 to 2 weight %.

The cyclic resin composition of the present invention can also be applied to a medical instrument which is an artificial kidney device of the dialysis type needing a high grade quatity guaranteed. Example of the quality standard is shown in the following:

### Artificial Kidney Device of Dialysis Type

The quality standard is according to Official Notifications Nos. 494 of the Japanese Ministry of Health and Welfare, Director of Drug Bureau.

A sanitary and high grade medical instrument which is an artificial kidney device of the dialysis type capable of satisfying the required quality standard can be obtained by forming the medical instrument or mechanical parts of the cyclic resin composition of the present invention, or laminating the same with the cyclic resin composition of the present invention.

A method of molding or laminating a medical instrument according to the present invention can be carried out by the known olefin resin molding techniques. For example, such a technique consists in heating the cyclic resin composition of the present invention by a screw, extruding into a metallic mold for a product, cooling and then taking the product out of the metallic mold. Also, the cyclic resin of the present invention may be heated and blown by an injection blow system, thus forming in a container, tube or mold for a medical instrument.

### Examples

Synthesis methods of the cyclic resin for use in the present invention and production processes of a medical instrument using the resin composition according to the present invention will be illustrated in detail without limiting the same.

### Synthetic Example 1 of Cyclic Resin: DCP Polymer (not in accordance with the invention)

3.6 liters of purified and dehydrated toluene and 1.2 kg of tricyclo[4,3,0,1^{2. 5}]-3,8-decene (DCP) were charged in a reactor of 10 liters, equipped with a stirrer, to which 72 g of triethylaluminum, 236 g of triethylamine and 62 g of titanium tetrachloride were added in a nitrogen atmosphere at 5 °C, and the mixture was heated to 25°C and stirred for 24 hours to effect polymerization. Then, the reaction was stopped by adding methanol and the resulting resin was precipitated with methanol, followed by washing with acetone-isopropyl alcohol (1 : 1) and drying in vacuum at a low temperature, thus obtaining 800 g of a polymer.

The thus obtained polymer was charged into an autoclave of 5 liters, equipped with a stirrer, in the form of a 10 weight % solution in cyclohexane, to which 25 g of palladium carbon was added in a hydrogen atmosphere, followed by replacing by hydrogen, raising the temperature to 120°C and supplementing hydrogen at a hydrogen pressure of 70 atm to effect hydrogenation for 12 hours. After the hydrogenation, the reaction mixture was subjected to centrifugal separation of the catalyst and then to precipitation in a large amount of a mixed solvent of acetone-isopropyl alcohol (1 : 1). To 100 parts by weight of the resulting resin were added 0.4 part by weight of BHT and 0.1 part by weight of Antigen TPS (commercial name), as an age resistor, thus obtaining 560 g of a resin (referred to as Resin (a)) having a softening point of 152°C and a bromine number of 0.2.

### Synthetic Example 2 of Cyclic Resin; DCP-Ethylene Copolymer

In a reaction vessel of 10 liters, equipped with a stirrer and dropping funnel, were charged 5 liters of purified and dehydrated talon and then 350 g of purified and dehydrated DCP, to which maintaining the temperature at lower than 3°C, 105 g of ethylaluminum sesquichloride and 110 g of dichloroethoxyoxovanadium, as a catalyst, were dropwise added while passing a mixed gas of dry ethylene and nitrogen gas (1 : 2) and stirring at a temperature of 20°C for 2 hours, thus effecting the polymerisation. The copolymerization was then stopped by the use of 30 ml of methanol. A copolymer was precipitated in methanol, washed with acetone and subjected to drying in vacuum at a low temperature, thus obtaining 312 g of a copolymer.

The thus obtained copolymer was charged in a 5-liter autoclave, equipped with a stirrer, in the form of a 10 weight % solution in cyclohexane, to which 25 g of palladium carbon was added in a hydrogen atmosphere, followed by replacing by hydrogen and raising the temperature to 120°C with agitation. Then, the hydrogen pressure was raised to 70 atm at the same temperature and hydrogen was supplemented at the same pressure to effect hydrogenation for 10 hours. After the hydrogenation, the reaction mixture was subjected to centrifugal separation of the catalyst and then to precipitation in a large amount of a mixed solvent of acetone-isopropyl alcohol (1 : 1), followed by filtering. To 100 parts by weight of the resulting copolymer were added 0.6 part by weight of BHT, followed by drying in vacuum, thus obtaining 300 g of a resin (referred to as Resin (b)) having a softening point of 146°C and a bromine number of 0.1.

### Synthetic Example 3 of Cyclic Resin: Copolymer of Bridged Polycyclic

### Hydrocarbon and Monocyclic Olefin

In a reaction vessel of 10 liters, equipped with a stirrer, were charged 4.5 liters of purified and dehydrated toluene and 300 g of mixed monomers of purified and dehydrated hexacyclo[6,6,1,1^{3. 6}, 1^{10.13}, O^{2. 7}, O^{9. 14}]-4-heptadecene and cyclopentene (1 : 1), to which 90 g of ethylaluminum sesquichloride and 15 g of dichloroethoxyoxovanadium were dropwise added in a nitrogen atmosphere at a temperature of at most 5°C. After raising the temperature to 10°C, the reaction mixture was stirred for 24 hours to effect the polymerisation. The polymerisation was then stopped by the use of 150 ml of methanol and the copolymer was precipitated in methyl, followed by washing and filtering. The copolymer resin was then hydrogenated in an analogous manner to Synthetic Example 2. 0.3 part by weight of Irganox 1076 (commercial name) was added to 100 parts by weight of the resulting copolymer, uniformly mixed and dried in vacuum to obtain 160 g of a resin (referred to as Resin (c)) having a softening point of 136-156°C and a bromine number of 0.2.

### Synthetic Example 4 of Cyclic Resin (not in accordance with the invention)

In a reaction vessel of 10 liters, equipped with a stirrer, were charged 5 liters of purified and dehydrated cyclohexane and 300 g of purified and dehydrated dimethyl-tetracyclo[4,4,0, 1^{2. 5}, 1⁷. ¹⁰]-3-dodecene, to which 20 g of dichloroethoxy-oxovanadium and 110 g of ethylaluminum sesquichloride were dropwise added in a nitrogen atmosphere at a temperature of at most 5°C. A mixed gases of nitrogen gas : hydrogen gas (150 : 1) was passed there through at a temperature of 10°C for 15 hours to effect polymerisation. The polymerisation was then stopped by the use of 1000 ml of isopropyl alcohol and the polymer was precipitated in isopropyl alcohol, followed by washing. 0.1 part by weight of Irganox 168 (commercial name) and 0.2 part by weight of lonox 330 (commercial name) were added to 100 parts by weight of the resulting polymer and dried in vacuum to obtain 182 g of a resin (referred to as Resin (d)) having a softening point of 141-150°C.

### Synthetic Example 5 of Cyclic Resin (not in accordance with the invention)

500 g of bis(methacryloxy)tricyclo(4,3,0, 1^{2. 5}]-decane and 500 g of cyclohexane were charged in a reactor of 5 liters, equipped with a stirrer, to which 30 g of benzoyl peroxide was added with passing nitrogen gas, followed by uniformly mixing and gradually heating to 120°C and effecting the polymerization reaction for 7 hours. After removing the solvent, 30 g of t-butylperoxybenzoate and 3 g of 4,4'-thiobis(6-t-butyl-3-methylphenol) were uniformly added, heated at a mold temperature of 170°C for 10 minutes to obtain a resinous powder and adequately washed with warm water, thus obtaining a resin (referred to as Resin (e)) having a softening point of at least 120°C and a bromine number of 4.3.

### Synthetic Example 6 of Cyclic Resin: Bridged (not in accordance with the invention)

### Polycyclic Compound

To a reactor of 2 liters, equipped with a stirrer, were charged 250 g of methyloxycarbonyltetracyclo[4,4,0,1^{2.} 5, 1^{7. 10}]-3-dodecene,1000 ml of 2-dichloroethane, 1.9 g of 1-hexene, 46 ml of a chlorobenzene solution of 0.05 mol/liter of tungsten exclude as a catalyst, 35 ml of a 1,2-dichloroethane solution of 0.1 mol/liter of paraldehyde and 19 ml of a toluene solution of 0.5 mol/liter of triisobutyl-aluminum in a nitrogen atmosphere, and the polymerisation was carried out at 60°C for 10 hours. 50 ml of methanol was added to the polymerisation system to stop the polymerisation, the solvent was evaporated and the product was washed with a mixed solution of acetone-methanol (1 : 1) and dried in vacuum. The polymerised product was dissolved in 4500 ml of tetrahydrofuran, to which 23 g of a palladium-alumina catalyst containing 5 weight % of palladium was added, and the mixture was then subjected to hydrogenation reaction at a temperature of 170°C and a hydrogen gas pressure of 100 kg/cm² for 5 hours. Then, the product was treated in an analogous manner to the treatment after the hydrogenation in Synthetic Example 1 of Cyclic Resin to obtain a polymerised resin. 0.5 part by weight of BHT was added to 100 parts by weight of the resin to obtain a resin (referred to as Resin (f)) having a softening point of at least 132°C and a bromine number of 0.05.

### Synthetic Example 7 of Cyclic Resin

In a reaction vessel of 10 liters, equipped with a stirrer, were charged 5 liters of purified and dehydrated toluene, to which 152 g of purified and dehydrated tetracyclo[4,4,0,1^{2. 5}, 1^{7.10}]-3-dodecene, 19 g of methylcyclohexene, and 18 g of ethylaluminum sesquichloride and 11 g of vanadium oxytrichloride were added and mixed in a nitrogen atmosphere at a temperature of at most 5°C. Mixed gases of dried ethylene gas : nitrogen gas (1 : 2) was passed there through from a gas feed pipe, the temperature was raised to 10°C and the polymerisation reaction was carried out using 15 liters of the mixed gas for 1 hour. The polymerization was then stopped by the use of 50 ml of methanol and the polymer was precipitated in a large amount of methyl, followed by washing with a mixed solvent of acetone-isopropyl alcohol (1 : 1). 0.3 part by weight of Irganox 1070 (commercial name) was added to 100 parts by weight of the resulting polymer to obtain a resin (referred to as Resin (g)) having a softening point of at least 124°C and a bromine number of 0.5.

### Synthetic Example 8 of Cyclic Resin (not in accordance with the invention)

In a reaction vessel of 10 liters, equipped with a stirrer, were charged 7 liters of purified and dehydrated toluene, to which 930 g of tetracyclo[4,4,0, 1^{2. 5},1^{7. 10}]-3-dodecene, 70 g of bicyclo[2,2,1]-2-heptoene, 5 g of 1-hexane, 6 g of tungsten hexachloride and 7 g of tetraphenyltin were added, followed by effecting the polymerisation at a temperature of 50°C for 3 hours. After the polymerisation, methanol was added thereto to precipitate a resin and the resin was washed with a mixed solution of acetone-methanol (1: 1) and dried in vacuum to obtain 980 g of a resin. The resulting resin was hydrogenated in an analogous manner to Synthetic Example 1, thus obtaining 930 g of a resin having a softening point of 155-160°C . During use of the resin, 0.5 weight % of BHT was added to 930 g of the resin (which will be referred to as Resin (h)).

### Synthetic Example 9 of Cyclic Resin

In a reaction vessel, equipped with a stirrer, were mixed 600 g of tetracyclo[4,4,0, 1^{2.5}, 1^{7.10}]-3-dodecene, 140 g of dicyclopentadiene, 180 g of pentacyclopentadecadiene, 760 g of 1-hexene and 2700 g of toluene, purified and dried, to which 18 g of triethylaluminum, 36 g of triethylamine and 5.5 g of titanium tetrachloride were added in the form of a solution in purified and dried toluene, while stirring, in a nitrogen atmosphere at a temperature of 25°C. The polymerisation was carried out at the same temperature for 5 hours. Acetone-isopropyl alcohol (1: 1) was added to the reaction vessel to stop the polymerisation and a resin was precipitated, filtered and dried in vacuum. Then, the resin was dissolved in 6000 ml of cyclohexane and subjected to hydrogenation by adding 60 g of palladium-carbon in a hydrogen gas atmosphere at a hydrogen pressure of 60 kg/cm² and a temperature of 155°C for 5 hours. The resin was filtered, precipitated in acetone-isopropyl alcohol (1: 1) and washed, followed by adding 0.2 weight % of BHT thereto and drying in vacuum to obtain 360 g of a resin (referred to as Resin (i)) having a softening point of 186°C.

### Examples 1 to 7 (not in accordance with the present invention) and Comparative Example 1 (not in accordance with the present invention)

Using Resins (a) to (g), obtained in Synthetic Examples of Cyclic Resins as described above, syringes with shapes of Fig. 2 were molded according to compositions and molding conditions as shown in Table 1 (Examples 1 to 7) (not in accordance with the present invention). **[0063]** For comparison, a syringe was similarly molded according to conditions shown in Table 1 using PP (Polypro 6200 E -commercial name- made by Mitsubishi Kasei KK). (Comparative Example 1) (not in accordance with the present invention).
- In Fig. 2,: 1: barrel; 2: plunger; 3: gasket; 4: cylinder nozzle 5: injection needle; 6: flange; 7: cyclic resin coating of the invention; 8: medicament liquid; 9: threaded engagement part; 10: injection needle cap

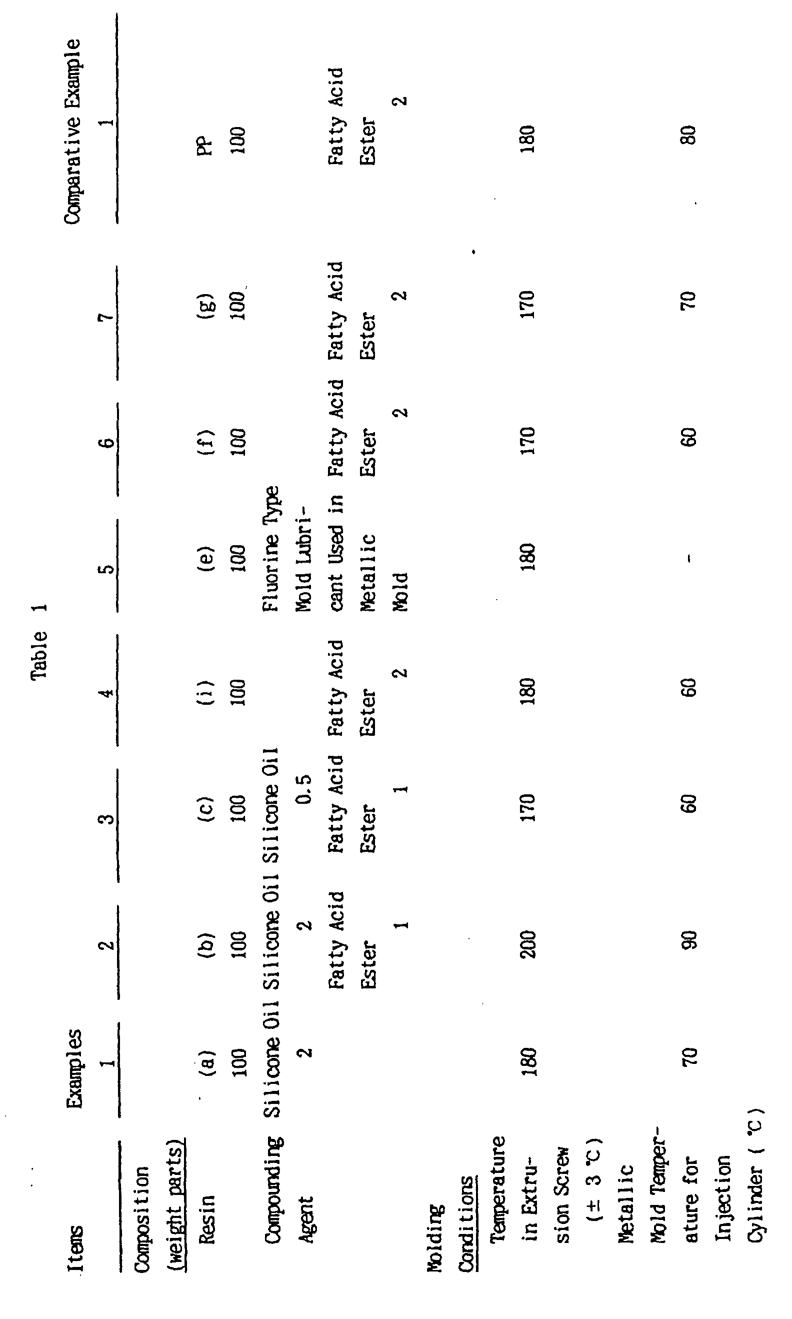

The syringe-cum-container holding a medicament liquid or distilled water for injection in its injection barrel was further subjected to a sanitary test according to "49 Test Method of Plastic Container for Liquid Transfusion" of JP 12, thus obtaining results as shown in Table 2 (not in accordance with the present invention).

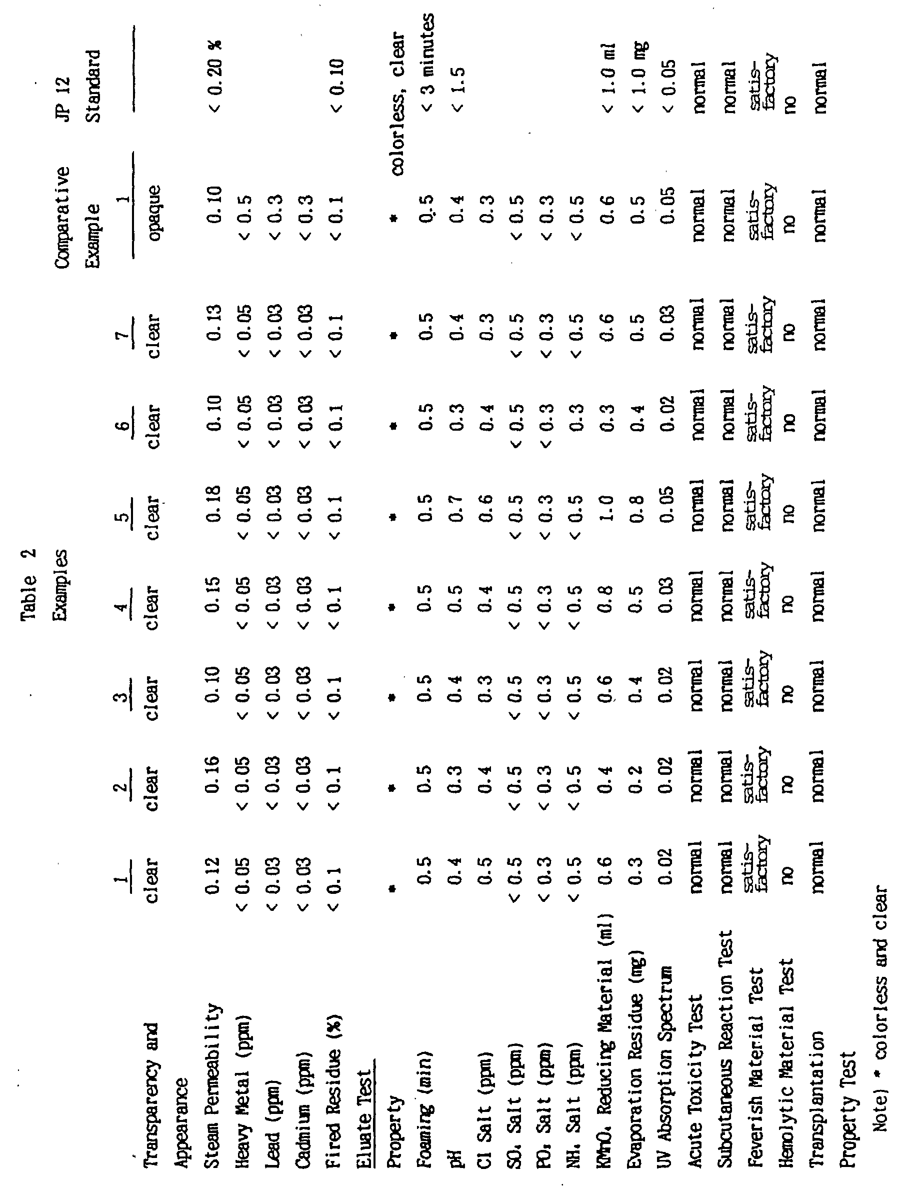

As shown in Table 2, the articles satisfy the standards and exhibit normal properties, but the article of Comparative Example 1 is opaque and is not suitable as a container for injection. When using commercially available resins, PVC or PE, a molded article deforms or adheres by a high pressure steam sterilization treatment at a temperature of 120°C for 60 minutes corresponding to the test conditions of JP 12. This means that such a comparative article is an unsatisfactory article. The molded articles of the cyclic resins of the present invention are transparent and do not deform even by the high pressure steam sterilization treatment.

The details of the tests shown in Table 2 are illustrated below:

### JP 12 Test

Property of Elution Test, Foaming, pH, Zn, KMnO4

### Reducing Property,

Evaporation Residue, UV (ultraviolet ray)

### Absorption Spectrum

A sample is mixed with water in an amount of 10 times as much as the sample and then heated and extracted with high pressure steam at 120°C for 1 hour. In view of that DIN or BS is carried out by heating at 121°C for 30 minutes, it is apparent that the extraction condition of JP 12 is the severest.

Acute Toxicity, Subcutaneous Reactions, Feverish Materials, Hemolytic Materials and Transplantation Tests:

These tests are carried out according to JP 12, which are somewhat different from those of DIN, BS or USP.

Since the article of the present invention is a transparent resin article, the article is suitable for showing foreign matter or crystalline matter in a medicament liquid flowing through the dripping tube or coupling tube. Bonding of the instruments or parts with each other can readily and strongly be effected.

As a test method of the chemical resistance of the synthetic resin, each of the instrument parts is immersed in a 0.5 weight % solution of sodium carbonate in an amount of 10 times as much as the weight of the each part, subjected to steam heating at a temperature of 121 °C for 30 minutes, and the solution is then subjected to measurement of the percent transmission of visible light with a wavelength of 430 nm and 650 nm, thus obtaining a percent transmission of at least 90 %. This means that the resin is excellent in chemical resistance.

An example of a medical instrument will now be illustrated (not in accordance with the present invention).

### Example 8 : Syringe-cum-Container Filled with Two Medicines in One Container (not in accordance with the present invention).

The mechanism of a syringe-cum-container (referred to as "container") filled with two kinds of medicines and formed of a cyclic resin is shown in Fig. 10, in which a high concentration powdered medicament 38 is charged in a cylinder nozzle 37 of an injection barrel 1 and a bypass groove 42 is positioned at the end of the middle part of the injection barrel. As shown in A-A'cross-sectional view of the bypass groove 42 of Fig. 11, the thickness of the injection cylinder is rendered uniform and the outer circumference of the cylinder is rendered convex, i.e. the inner wall is enlarged at the middle part thereof to form the groove 42. A first sealing stopper 39 is substantially positioned at the middle part of the injection barrel 1 in the container, which is so designed that the thickness of the first sealing stopper 39 is less than the length of the bypass groove 42 and the stopper is completely embraced by the groove, as shown by broken line in Fig. 10.

A second sealing stopper 40 is positioned at the aperture of the injection barrel 1, the second sealing stopper 40 having a hollow into which the end of a plunger 2 is to be inserted, and distilled water or a dilute medicamnet liquid 41 is charged in between the second sealing stopper 40 and first sealing stopper 39. The aperture of the barrel 1 is provided with a flange 6 to be covered by a cap 10 to close the aperture. The nozzle end 37 of the injection barrel 1 is narrowly elongated to give a shape 43 of an injection needle and the point is closed by a rubber cap 10.

When using a medicament container of this Example, the end of the push rod is connected with the second sealing stopper in the injection barrel and the plunger is forced in the injection barrel to push the distilled water and first sealing stopper. When the first sealing stopper is thus positioned in the bypass groove, the distilled water is caused to flow through the bypass groove, mixed with the powdered medicament 38 and dissolves the powdered medicament to form a liquid medicament with a concentration to be dosed. The thus diluted medicament liquid is dosed by the octopus tube 17 as shown in Fig. 3 in many cases.

Production of the injection barrel was generally carried out by heating and melting at a temperature of 230 to 300°C firstly an amorphous nylon (Novamid X 21 -commercial name- made by Mitsubishi Kasei Kogyo KK) and secondly the cyclic resin (f), in a metallic mold of an injection barrel type, subjecting to injection molding and then cooling.

The first sealing stopper and second sealing stopper were prepared by blending 100 parts by weight of BR (BR 01 -commercial name- made by Japan Synthetic Rubber Co., Ltd.) and 20 parts by weight of ultra-high molecular weight polyethylene powder (Hizex Million 240 -commercial name- made by Mitsui Sekiyu Kagaku KK) as a raw material rubber, crosslinked by using an organoperoxide to adjust the hardness of the product to 48 ± 5 and annular protrusions were formed on the surface of the rubber stopper.

When the syringe-cum-container was further subjected to a test according to "49 Test Method of Plastic Container for Liquid Transfusion" of JP 12, it exhibited a value inside the test standard value and passed the standard.

The benefits or effects of the present invention are summarized below:
(1) Since the medical instrument of the present invention is formed of the specified cyclic resin, medicament liquids such as pharmaceuticals and nutriments can be maintained in high quality and dosed correctly and sanitarily.
(2) The cyclic resin is inert to acid or alkali solutions and meets with less stripping of fine particles, etc. from the surface of the medical instrument and less adsorption of medicament liquids on the surface thereof, thus having a good sanitary property.
(3) Influences upon medicaments by individual or overall external factors i.e. environments such as heat, oxygen, air, humidity (moisture), light (ultraviolet rays) and the like, and outer forces can be reduced.
(4) The medical instrument of the present invention stands the test standards for medical instruments provided by JP 12 and Official Notification of the Ministry of Health and Welfare (Japan).
(5) Molding of the medical instrument of the present invention can readily be accomplished.

Thus, the present invention provides a sanitary medical instrument having the foregoing features and which is therefore of great benefit.

## Claims

1. A medical instrument consisting of a material containing a resin formed of a bridged polycyclic hydrocarbon compound, as a polymeric component,
said medical instrument being
an artificial kidney device of the dialysis type,
wherein:
(a) the bridged polycyclic hydrocarbon compound has at least one unsaturated bond in the ring or a substituent thereof;
(b) the resin contains at least one member selected from lower olefins, aromatic compounds or vinyl monomers of lower olefins or aromatic compounds, as a copolymeric component;
(c) the resin is a mixture with at least one member selected from olefinic resins and synthetic rubbers, the resin being present in a proportion of at least 30% by weight; and
(d) the resin has a bromine number of at most 1 and a softening point of at least 90°C.

## Patentansprüche

1. Medizinisches Instrument, das aus einem Material besteht, das ein Harz enthält, das aus einer verbrückten polycyclischen Kohlenwasserstoffverbindung als polymerer Komponente ausgebildet ist, wobei das medizinische Instrument eine künstliche Niere vom Dialyse-Typ ist, wobei
(a) die verbrückte polycyclische Kohlenwasserstoffverbindung mindestens eine ungesättigte Bindung in dem Ring oder einem Substituenten davon aufweist;
(b) das Harz mindestens eine Komponente enthält, die aus Niederolefinen, aromatischen Verbindungen, und Vinylmonomeren von Niederolefinen oder aromatischen Verbindungen, als copolymere Komponente ausgewählt ist;
(c) das Harz als Mischung mit mindestens einer Komponente vorliegt, die aus Olefinharzen und Synthesekautschuken ausgewählt ist, wobei das Harz in einem Anteil von mindestens 30 Gew.-% vorhanden ist, und
(d) das Harz eine Bromzahl von höchstens 1 und einen Erweichungspunkt von mindestens 90°C besitzt.

## Revendications

1. Instrument médical constitué d'un matériau contenant une résine formée d'un composé hydrocarboné polycyclique ponté, en tant que composant polymère, ledit instrument médical étant un appareillage de rein artificiel de type dialyse, dans lequel:
(a) le composé hydrocarboné polycyclique ponté a au moins une liaison insaturée dans le noyau ou un substituant de celui-ci;
(b) la résine contient au moins un élément choisi parmi des oléfines inférieures, des composés aromatiques et des monomères vinyliques d'oléfines inférieures ou de composés aromatiques, en tant que composant copolymère;
(c) la résine est un mélange avec au moins un élément choisi parmi des résines oléfiniques et des caoutchoucs synthétiques, la résine étant présente dans une proportion d'au moins 30% en poids; et
(d) la résine a un indice de brome d'au plus 1 et un point de ramollissement d'au moins 90°C.
